# EUROPÄISCHE PATENTANMELDUNG

(11) **EP 4 810 836 A1**
(43) Veröffentlichungstag der Anmeldung: **23.09.2026**
(21) Anmeldenummer: 25164241.9
(22) Anmeldetag: 17.03.2025
(51) Int. Cl.: F16K 7/04, F16K 31/08, A61M 39/28

(54) **SCHLAUCHKLEMMVORRICHTUNG UND BEDIENVERFAHREN**

(71) Anmelder: Kendrion Kuhnke Automation GmbH, 23714 Malente (DE)
(72) Erfinder: Nuppenau, Lukas, 22941 Bargteheide (DE); Dohse, Inga, 23714 Malente (DE); Schnabel, Burghart, 24217 Kiel (DE); Kühn, Volker, 24790 Schacht-Audorf (DE); Paap, Jannes, 24149 Kiel (DE); Stegelmann, Jochen, 23684 Gronenberg (DE)
(74) Vertreter: Seemann & Partner Patentanwälte mbB

(57) **Zusammenfassung**

Die Erfindung betrifft eine Schlauchklemmvorrichtung (10) mit einem Gehäuse (16) und einer außerhalb des Gehäuses (16) angeordneten und in ihrem Querschnitt zwischen einer Klemmposition und einer geöffneten Position reversibel veränderbaren Schlauchdurchführung (12), die von einem fixen Gegenlager (14) und einem gegenüber dem fixen Gegenlager (14) verschiebbaren Druckstück (20) gebildet wird, welches mit einem in Richtung auf die Klemmposition federvorbelasteten Anker (28) eines Elektromagneten (26) verbunden ist, welcher im bestromten Zustand des Elektromagneten (26) das Druckstück (20) entgegen der Federvorbelastung vom Gegenlager (14) entfernt und dadurch die Schlauchdurchführung (12) aus der Klemmposition in die geöffnete Position bringt, sowie ein Verfahren zum Bedienen einer Schlauchklemmvorrichtung.

Erfindungsgemäß ist das Druckstück (20) mit dem Anker (28) über einen Adapter (24) verbunden, mittels dessen eine in der Klemmposition der Schlauchdurchführung (12) eingenommene Spaltbreite zwischen Druckstück (20) und dem fixen Gegenlager (14) einstellbar ist.

## Beschreibung

Die Erfindung betrifft eine Schlauchklemmvorrichtung mit einem Gehäuse und einer außerhalb des Gehäuses angeordneten und in ihrem Querschnitt zwischen einer Klemmposition und einer geöffneten Position reversibel veränderbaren Schlauchdurchführung, die von einem fixen Gegenlager und einem gegenüber dem fixen Gegenlager verschiebbaren Druckstück gebildet wird, welches mit einem in Richtung auf die Klemmposition federvorbelasteten Anker eines Elektromagneten verbunden ist, welcher im bestromten Zustand des Elektromagneten das Druckstück entgegen der Federvorbelastung vom Gegenlager entfernt und dadurch die Schlauchdurchführung aus der Klemmposition in die geöffnete Position bringt. Die Erfindung betrifft außerdem ein Verfahren zum Bedienen einer Schlauchklemmvorrichtung.

Schlauchklemmvorrichtungen zum Klemmen von Schläuchen, insbesondere in medizinischen Anwendungen wie beispielsweise der Dialyse, sind bekannt. Solche Schlauchklemmvorrichtungen verfügen über einen Elektromagneten, der angesteuert wird, um eine Durchgangsöffnung für einen Schlauch, eine sogenannte Schlauchdurchführung, zu öffnen oder so weit zu schließen, dass jeglicher Durchfluss von Flüssigkeiten oder Gasen durch den Schlauch abgeklemmt wird. Dazu wird üblicherweise ein Druckstück mit einer im Profil keilförmigen Kante mit einer definierten Klemmkraft auf ein fixes Gegenlager zubewegt, bis eine minimale Spalthöhe des Klemmspalts der Schlauchdurchführung erreicht ist. Da der Schlauch selbst nicht beschädigt werden darf, gibt es für verschiedene Schlauchdurchmesser verschieden dimensionierte Schlauchklemmvorrichtungen. Mehrere solcher Schlauchklemmvorrichtungen für unterschiedliche Schlauchgrößen können in ein Gerät eingebaut sein, so dass jeweils nur ein kleiner Teil mit der jeweiligen Schlauchdurchführung nach außen ragt und zugänglich ist, während der Teil mit dem Elektromagneten im Inneren des Geräts angeordnet ist.

Die Bedienung einer Schlauchklemmvorrichtung kennt im Wesentlichen drei Betriebszustände, nämlich das Einlegen des Schlauches, den Normalbetrieb und den Notbetrieb. Zum Einlegen des Schlauches wird die Schlauchdurchführung geöffnet, in dem die Magnetspule bestromt wird, der Anker in die Magnetspule gezogen wird und dabei das starr mit dem Anker verbundene Druckstück mitnimmt, wodurch sich der Klemmspalt öffnet. Im Normalbetrieb ist der Schlauch in die Schlauchdurchführung eingelegt und die Magnetspule wird bestromt, wenn ein Fluid durch den Schlauch fließen soll, und nicht bestromt, wenn der Schlauch abgeklemmt werden soll. Im Falle einer Fehlfunktion, d. h. eines Stromausfalls beispielsweise, soll der Standardzustand sein, dass der Schlauch abgeklemmt ist. Aus diesem Grund wird die Schlauchklemmvorrichtung immer so eingerichtet, dass zum Öffnen ein elektrischer Strom durch die Magnetspule des Elektromagneten notwendig ist. Dies führt allerdings dazu, dass im Notfall, wenn kein Strom zur Bestromung der Magnetspule zur Verfügung steht, der Schlauch standardmäßig abgeklemmt ist. Daraus ergibt sich der Notbetrieb, bei dem auch im Falle eines Stromausfalls der Schlauch entnommen werden kann, in dem eine Notentriegelung vorgesehen wird, bei der das Druckstück mechanisch bzw. händisch betätigt wird, um den Klemmspalt zu öffnen.

Für die Schlauchdurchführungen bekannter Schlauchklemmvorrichtungen gibt es zur Sicherung des Schlauches verschiedene Lösungen. Es gibt Schlauchklemmvorrichtungen mit vollständig umschlossenen Schlauchdurchführungen. Diese bieten sicheren Halt für die Schläuche und schützen davor, dass diese im geöffneten Zustand aus der Schlauchdurchführung herausrutschen. Allerdings müssen die Schläuche durch eine solche Schlauchdurchführung hindurchgefädelt werden, was nicht immer ganz einfach ist.

Es gibt auch zu einer Seite offene Schlauchdurchführungen, bei denen das Einlegen des Schlauchs sehr einfach ist. Jedoch besteht bei diesen Schlauchdurchführungen die Gefahr, dass der Schlauch im geöffneten Zustand der Schlauchdurchführung seitlich herausrutschen kann, wenn er beispielsweise unter mechanischer Spannung steht. Manche einseitig offenen Schlauchdurchführungen haben in der Kontur des Gegenlagers, die dem Druckstück gegenüberliegt, eine halbkreisförmige Aussparung, welche den Schlauch zurückhalten kann, wobei die Größe der Aussparung für den Schlauchtyp dimensioniert ist, für den die Schlauchklemmvorrichtung bestimmt ist.

Einige der bei bekannten Schlauchdurchführungen auftretenden Probleme liegen neben der Sicherung von Schläuchen gegen seitliches Herausrutschen auch in der Notwendigkeit, eine Vielzahl von Schlauchklemmvorrichtungen für verschiedene Schlauchdurchmesser bereit zu halten. Die Druckstücke dieser Schlauchklemmvorrichtungen haben jeweils konstante Hübe und neigen zum Verkanten, was zu einer Verkürzung der Lebensdauer beiträgt.

Der vorliegenden Erfindung liegt die Aufgabe zugrunde, eine Schlauchklemmvorrichtung sowie ein Verfahren zu dessen Bedienung zur Verfügung zu stellen, die einige der Nachteile der bekannten Schlauchklemmvorrichtungen überwinden.

Diese Aufgabe wir durch eine Schlauchklemmvorrichtung mit einem Gehäuse und einer außerhalb des Gehäuses angeordneten und in ihrem Querschnitt zwischen einer Klemmposition und einer geöffneten Position reversibel veränderbaren Schlauchdurchführung, die von einem fixen Gegenlager und einem gegenüber dem fixen Gegenlager verschiebbaren Druckstück gebildet wird, welches mit einem in Richtung auf die Klemmposition federvorbelasteten Anker eines Elektromagneten verbunden ist, welcher im bestromten Zustand des Elektromagneten das Druckstück entgegen der Federvorbelastung vom Gegenlager entfernt und dadurch die Schlauchdurchführung aus der Klemmposition in die geöffnete Position bringt, gelöst, welche dadurch weitergebildet ist, dass das Druckstück mit dem Anker über einen Adapter verbunden ist, mittels dessen eine in der Klemmposition der Schlauchdurchführung eingenommene Spaltbreite zwischen Druckstück und dem fixen Gegenlager einstellbar ist.

Mit dem Adapter ist somit nunmehr möglich, den Klemmspalt einzustellen und auf diese Weise die Schlauchklemmvorrichtung für verschiedene Schlauchgrößen einzustellen und zu verwenden. Dies ermöglicht es, eine kleinere Menge von Schlauchklemmvorrichtungen als bisher vorzuhalten, weil nicht mehr für jede Schlauchgröße eigene Schlauchklemmvorrichtungen eingesetzt werden müssen, die vorrätig gehalten werden müssen. Der Adapter funktioniert im Wesentlich so, dass er den Abstand des Druckstücks vom Anker verändert, so dass das Klemmspaltmaß bei gleichbleibendem Hub des Ankers verändert werden kann. Dies kann beispielsweise durch eine Schraube-Mutter-Verbindung erfolgen, bei der eine Mutter, auf der ein Stützkörper aufsetzt, auf dem das Druckstück abgestützt wird, auf der mit dem Anker verbundenen Schraube in ihrer Position verstellt werden kann. Ebenfalls können Klemmverbindungen verwendet werden, bei denen ein Halteelement auf einem mit dem Anker verbundenen Bolzen verschoben werden können.

In Ausführungsformen der Schlauchklemmvorrichtung ist der Adapter im montierten Zustand der Schlauchklemmvorrichtung einstellbar und insbesondere durch eine Öffnung im Gehäuse zugänglich. Die Einstellung kann beispielsweise so erfolgen, dass der Adapter mit dem Anker des Elektromagneten über eine Schraubverbindung verbunden ist und durch Drehung relativ zum Anker in seinem Abstand zum Anker verstellbar ist. In Ausführungsformen kann der Adapter durch eine Öffnung im Gehäuse mittels eines geeigneten Instruments erfasst und gehalten werden und gleichzeitig kann der Anker mit einem unteren Teil des Bolzens um seine Achse gedreht werden, so dass sich der Abstand des nicht mitrotierenden Adapters vom Anker vergrößert oder verkleinert. Der Adapter kann auch so ausgeführt und angeordnet sein, dass er von einem formkomplementären Teil im Inneren des Gehäuses daran gehindert wird, sich mit dem Bolzen mitzudrehen. In dem Fall ist keine Öffnung im Gehäuse notwendig, durch die der Adapter erfasst wird. Umgekehrt kann in anderen Ausführungsformen auch der Adapter gedreht werden, während der Anker in seiner Position verbleibt. Die Einstellung kann als letzter Montageschritt erfolgen, was erheblich Werkzeug- und Entwicklungskosten einspart, jedoch ist auch eine nachträgliche Änderung möglich. Die Klemmspaltgröße kann so auf eine neue Schlauchgröße eingestellt werden, ohne dass die Schlauchklemmvorrichtung aus dem Gerät herausgenommen werden muss, in welchem sie montiert ist. Damit für ausreichende Hygiene gesorgt.

In einer weiteren Ausführungsform ist das Druckstück mit dem Anker flexibel verbunden und/oder vom Anker entkoppelt. Dies ergibt ein tolerantes Design, welches bei einer möglichen Fehleinstellung des Klemmspaltes bezüglich eines Schlauchdurchmessers übermäßige Kräfte ableiten kann, so dass es nicht zu einer Beschädigung des Schlauchs kommt. Die flexible Verbindung kann auch Beschädigungen dadurch verhindern, dass sich das Druckstück während des Hubs verkantet. Die Verbindung des Druckstücks mit dem Anker kann beispielsweise so ausgebildet sein, dass sie über ein oder mehrere Zwischenstücke erfolgt, welche übermäßige Kräfte beispielsweise in elastischer Weise durch reversible Verformung zerstörungsfrei aufnehmen oder bei Überschreiten einer Grenzkraft eine unelastische Verformung erleiden und auf diese Weise dafür sorgen, dass die übermäßige Kraft vom Schlauch ferngehalten wird. In einer Ausführungsform kann sich auf dem Umfang des Adapters ein zusätzliches, elastisches Element befinden, welches Schiefstände zwischen Adapter und Druckstück zulässt. Die Kraftübertragung für das Klemmen eines Schlauchs im Klemmspalt erfolgt über einen Formschluss zwischen Adapter und Druckstück, auch im Falle eines Schiefstandes, damit die erforderlichen Kräfte sicher übertragen werden können. Der Rückhub beim Öffnen des Spaltes erfolgt dann über das elastische Element auf dem Umfang des Adapters.

In Ausführungsformen ist das fixe Gegenlager auf einer Seite der Schlauchdurchführung mit dem Gehäuse verbunden und weist zur anderen Seite eine seitliche Öffnung zum Einfädeln oder zur Entnahme eines zu klemmenden Schlauchs auf, wobei an der geöffneten Seite der Schlauchdurchführung ein Druckknopf angeordnet ist, welcher in einem Ruhezustand die seitliche Öffnung der Schlauchdurchführung verschließt und in einem um einen Hubweg gedrückten Zustand freigibt. Damit wird das Problem angegangen, dass eine Aussparung im fixen Gegenlager nur für Schläuche eines bestimmten Durchmessers einen zuverlässigen Schutz gegen ein seitliches Herausrutschen aus der Schlauchdurchführung bieten kann. Stattdessen wird die seitliche Öffnung mittels des Druckknopfes reversibel verschlossen.

Zum Einlegen eines Schlauches in die Schlauchdurchführung wird der Elektromagnet bestromt, so dass das Druckstück vom fixen Gegenlager abrückt und den Klemmspalt öffnet. Zusätzlich wird der Druckknopf so weit eingedrückt, bis die seitliche Öffnung der Schlauchdurchführung freigegeben ist und dann der Schlauch von der Seite in die Schlauchdurchführung eingeführt werden kann. Nachdem der Schlauch eingeführt ist, wird der Druckknopf wieder losgelassen, so dass er in seine Ruheposition zurückkehrt, in der er die seitliche Öffnung der Schlauchdurchführung verschließt. Der Schlauch ist nun daran gehindert, seitlich aus der Schlauchdurchführung herauszurutschen. Außerdem kann die Bestromung des Elektromagneten beendet werden, so dass das Druckstück den Schlauch klemm und jeglichen Durchfluss eines Fluids durch den Schlauch unterbindet.

In einer Weiterbildung, die einen Notbetrieb der Schlauchklemmvorrichtung erlaubt, ist der Druckknopf mit einem im Inneren des Gehäuses angeordneten Hebel an einem ersten Ende des Hebels verbunden, welches sich mit dem Hub des Druckknopfs mitbewegt, während ein zweites Ende des Hebels mit einem Gelenk im Gehäuse verbunden ist, wobei der Hebel bei Betätigung des Druckknopfes im unbestromten Zustand des Elektromagneten den Anker unmittelbar oder mittelbar kontaktiert und entgegen der Federvorbelastung des Ankers drückt, um die Schlauchdurchführung in die geöffnete Position zu bewegen. Dies ermöglicht es somit, den Druckknopf nicht nur zur Freigabe der seitlichen Öffnung zu verwenden, sondern im Notbetrieb zusätzlich zum Öffnen des Klemmspalts, falls der Elektromagnet unbestromt ist. Da dies der Federvorbelastung des Ankers entgegenwirkt, mit dem das Druckstück verbunden ist, erfolgt diese weitere Bewegung des Druckknopfes unter Aufwendung einer größeren Kraft als derjenigen, die im Normalbetrieb zum Freigeben der seitlichen Öffnung aufgewendet wird.

In Ausführungsformen weist der Druckknopf in der Richtung der seitlichen Ausdehnung der Schlauchdurchführung eine Breite auf, die größer ist als ein maximaler Abstand zwischen einer Klemmkontaktseite des Druckstücks und einer der Klemmkontaktseite des Druckstücks gegenüberliegenden Klemmkontaktseite des fixen Gegenlagers. Diese breite Ausführung ermöglicht es, den Druckknopf händisch zu betätigen und gleichzeitig einen Abstand der Hand zur seitlichen Öffnung der Schlauchdurchführung freizulassen, die es erlaubt, einen Schlauch aus der Schlauchdurchführung zu entnehmen oder in die Schlauchdurchführung einzuführen.

Vorteilhafterweise ist der Druckknopf durch eine Rückstellfeder in eine die seitliche Öffnung der Schlauchdurchführung verschließende Position federvorbelastet.

In Ausführungsformen wird die Federvorbelastung des Ankers durch eine Klemmfeder hergestellt, die auf der dem Druckstück abgewandten Seite des Ankers angeordnet ist. Die Klemmfeder kann sich auf einem Gegenlager abstützen, das an der Magnetspule oder dem Joch des Elektromagneten befestigt ist. Die Klemmfeder kann beispielsweise als Spiralfeder bzw. Druckfeder ausgebildet sein.

Die der Erfindung zugrundeliegende Aufgabe wird auch durch ein Verfahren zum Bedienen einer zuvor beschriebenen erfindungsgemäßen Schlauchklemmvorrichtung gelöst, wobei eine minimale Größe eines Spalts zwischen dem Druckstück und dem fixen Gegenlager der Schlauchklemmvorrichtung mittels des Adapters eingestellt wird. Dieses Verfahren verwirklicht die gleichen Vorteile, Eigenschaften und Merkmale wie die Schlauchklemmvorrichtung der vorliegenden Offenbarung.

In einer Weiterbildung wird der Druckknopf zum Freigeben der seitlichen Öffnung der Schlauchdurchführung betätigt und zum Verschlie-βen der seitlichen Öffnung losgelassen und kann insbesondere zum Notöffnen der Schlauchdurchführung weiter betätigt werden, als es zum Freigeben der seitlichen Öffnung der Schlauchdurchführung notwendig ist.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine bekannte Schlauchklemmvorrichtung in schematischer perspektivischer Darstellung,
- Fig. 2: die Schlauchklemmvorrichtung aus Fig. 1 im Querschnitt,
- Fig. 3: ein Ausführungsbeispiel einer Schlauchklemmvorrichtung gemäß der vorliegenden Offenbarung in schematischer perspektivischer Darstellung,
- Fig. 4: die Schlauchklemmvorrichtung aus Fig. 3 im Querschnitt,
- Fig. 5: Einzelheiten der Schlauchklemmvorrichtung in einer perspektivischen Ansicht,
- Fig. 6: zentrale Komponenten der Schlauchklemmvorrichtung im Querschnitt und
- Fig. 7: eine perspektivische Darstellung eines Ausführungsbeispiels eines Adapters.

In den Zeichnungen sind jeweils gleiche oder gleichartige Elemente und/oder Teile mit denselben Bezugsziffern versehen, so dass von einer erneuten Vorstellung jeweils abgesehen wird.

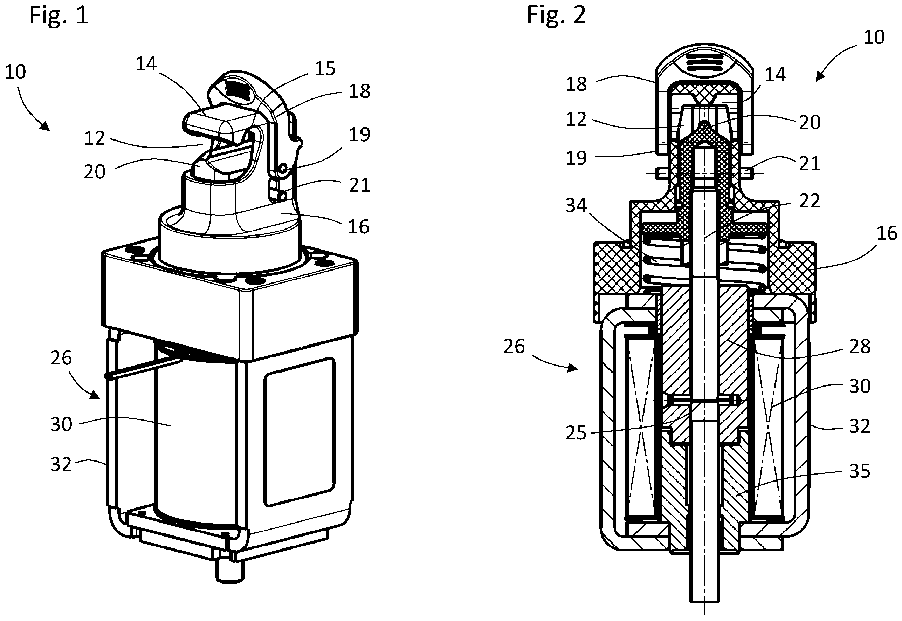

Fig. 1 zeigt eine bekannte Schlauchklemmvorrichtung 10 für eine bestimmte Schlauchgröße in schematischer perspektivischer Darstellung. An ihrer Spitze weist sie eine Schlauchdurchführung 12 auf, welche von verschiebbaren Druckstück 20 mit keilförmigem Profil einerseits und von einem Gegenlager 14 begrenzt wird. Das Gegenlager 14 ist an einer Seite der Schlauchdurchführung 12 mit einem Gehäuse 16 der Schlauchklemmvorrichtung 10 verbunden bzw. aus einem Stück mit dem Gehäuse 16. Zu einer Seite hin ist die Schlauchdurchführung 12 offen, um einen Schlauch (nicht dargestellt) in die Schlauchdurchführung 12 einführen oder aus dieser herausnehmen zu können. Weiterhin hat die Schlauchdurchführung 12 an der Unterseite des Gegenlagers 14 eine halbkreisförmige Aussparung 15, die dazu dient, den Schlauch davor zu schützen, seitlich durch die Öffnung wieder aus der Schlauchdurchführung 12 herauszurutschen. Die Aussparung 15 ist an den Durchmesser des Schlauchtyps angepasst, für den die Schlauchklemmvorrichtung 10 bestimmt ist.

Ferner weist die Schlauchklemmvorrichtung 10 einen Notentriegelungshebel 18 auf, welcher auf einem Scharnier 19 kippbar auf dem Gehäuse 16 aufsitzt. Im Notfall wird der Notentriegelungshebel 18 nach hinten weggeklappt. Die seitliche Öffnung der Schlauchdurchführung 12 bleibt immer offen. Der untere Teil des Notentriegelungshebels 18 ist mit einer Exzenterkulisse versehen, welche bei Betätigung des Notentriegelungshebels 18 einen mit dem Druckstück 20 verbundenen Mitnehmerbolzen 21 kontaktiert. Bei weiterer Betätigung drückt die Exzenterkulisse den Mitnehmerbolzen 21 und das damit verbundene Druckstück 20 herunter, um den die Schlauchdurchführung 12 bildenden Klemmspalt zu öffnen. Ein in der Schlauchdurchführung 12 positionierter Schlauch kann dann entnommen werden.

Wenn die Schlauchklemmvorrichtung 10 in einem Gerät mit mehreren Schlauchklemmvorrichtungen verbaut ist, ragt der obere Teil der Schlauchklemmvorrichtung 10 aus dem Gerät heraus. Der größere untere Teil ist im Gerät verborgen und von außen nicht zugänglich. Er umfasst einen weiteren Teil des Gehäuses 16 sowie einen Elektromagneten 26 der zur Verschiebung des Druckstücks 20 aus der Klemmposition in eine geöffnete Position dient. Die Magnetspule 30 und Joch 32 des Elektromagneten 26 sind in Fig. 1 abgebildet.

Fig. 2 zeigt Details der Schlauchklemmvorrichtung 10 aus Fig. 1 im Querschnitt. Gut erkennbar ist die im Querschnitt keilförmige Form des Druckstücks 20, ebenso, dass in diesem Ausführungsbeispiel das Gegenlager 14 im Querschnitt ebenfalls im Wesentlichen keilförmig ausgebildet ist. Das Druckstück 20 weist an seiner Unterseite eine flächige Erweiterung auf, gegen die von unten eine Klemmfeder 34 drückt, welche sich auf dem Joch 32 des Elektromagneten 26 abstützt.

Das Druckstück 20 ist über eine starre Druckstange 22 fest mit dem Anker 28 des Elektromagneten 26 verbunden. In ihrem oberen Bereich weist die Druckstange 22 einen querliegenden Mitnehmerbolzen 21 auf, der Teil des Notentriegelungssystems ist, welches im Zusammenhang mit Fig. 1 beschrieben worden ist. Wenn die Exzenterkulisse im unteren Bereich des Notentriegelungshebels 18 auf den Mitnehmerbolzen 21 trifft, drückt sie diesen nach unten und bewegt dadurch die Druckstange 22 und das Druckstück 20 nach unten, sodass sich die Schlauchdurchführung 12 öffnet.

In Fig. 2 ist zusätzlich zu erkennen, dass der Elektromagnet 26 als Zentralbestandteile von außen nach innen ein Joch 32, die Magnetspule 30 und den Anker 28 aufweist. Ferner ist innerhalb der Magnetspule 32 ein Lager 35 vorhanden, welches einen unteren Anschlag bietet und so die Eindringtiefe des Ankers 28 in die Magnetspule 30 begrenzt. Ferner ist der Fig. 2 zu entnehmen, dass der Anker 28 über einen Splint 25 mit der starren Druckstange 22 verbunden ist.

Der in den Figuren 1 und 2 gezeigte Zustand ist der Zustand, in dem die Magnetspule 30 bestromt ist und den Anker 28 in die Magnetspule 30 hinein bis zum Anschlag auf dem Lager 35 zieht. Über die starre Druckstange 22 wird das Druckstück 20 mitgezogen und so die Schlauchdurchführung 12 geöffnet. Dies erfolgt entgegen der durch die Klemmfeder 34 auf die Unterseite des Druckstücks 20 ausgeübte Kraft. Sobald die Magnetspule 30 nicht mehr bestromt wird, wirkt keine Kraft der Federkraft der Klemmfeder 34 mehr entgegen und diese drückt das Druckstück 20 mit ihrer definierten Federkraft nach oben, um den Klemmspalt der Schlauchdurchführung 12 zu verkleinern. Dieser Hub wird dadurch begrenzt, dass die Oberseite der flächigen Erweiterung an der Unterseite des Druckstücks 20 an die Oberseite des Gehäuses 16 stößt. Diese Begrenzungsflächen und der Anschlag am Lager 35 definieren den Hub, den das Druckstück 20 der Schlauchklemmvorrichtung 10 zwischen der Klemmstellung und der offenen Stellung ausführt.

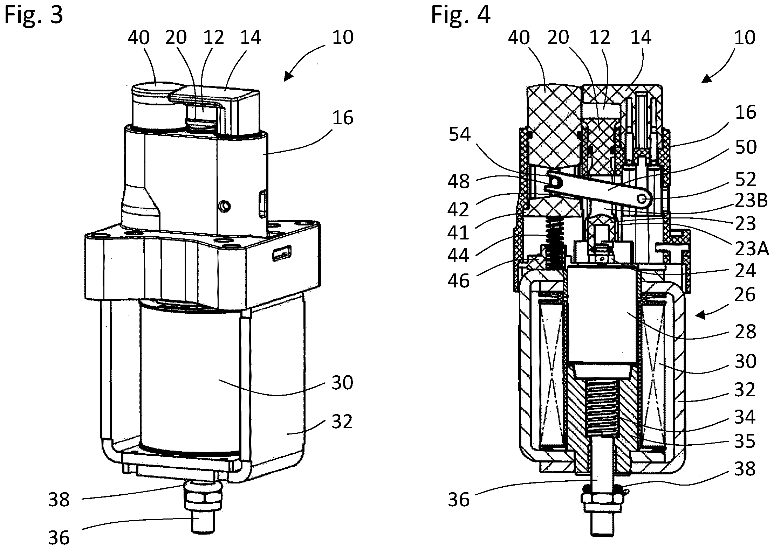

Fig. 3 zeigt ein Ausführungsbeispiel einer Schlauchklemmvorrichtung 10 gemäß der vorliegenden Offenbarung in schematischer perspektivischer Darstellung. Die offensichtlichsten äußerlichen Unterschiede zu der bekannten Schlauchklemmvorrichtung 10 aus Fig. 1 finden sich im Bereich der Schlauchdurchführung 12. Während das Druckstück 20 in seinem oberen Teil ähnlich aufgebaut ist wie dasjenige aus Fig. 1, bildet das Gegenlager 14 in diesem Fall ein "L"-förmiges Teil ohne eine halbkreisförmige Aussparung. Ein Schenkel des "L" des Gegenlagers 14, welches dem Druckstück 20 direkt gegenüberliegt, bildet die obere Begrenzung der Schlauchdurchführung 12. Dieser Teil ist im Querschnitt ebenfalls grob keilförmig. Der andere Schenkel des Gegenlagers 14 bildet die Verbindung zum Gehäuse 16 und eine seitliche Begrenzung der Schlauchdurchführung 12.

Zur anderen Seite hin ist die Schlauchdurchführung 12 offen. Sie wird von einem Druckknopf 40 begrenzt, welcher das seitliche offene Ende der Schlauchdurchführung 12 verschließt. Der Druckknopf 40 kann betätigt werden, in dem ein Druck auf die Oberfläche des Druccknopfs 40 ausgeübt wird. Der Druckknopf 40 versinkt dann im Gehäuse 16 und gibt die seitliche Öffnung der Schlauchdurchführung 12 frei. Dabei ist der Druckknopf 40 in dieser Ausführungsform so breit ausgeführt, dass er an seinem von dem Gegenlager 14 abgewandten Ende berührt werden kann, sodass ein Freiraum verbleibt, durch den ein Schlauch seitlich in die Schlauchdurchführung 12 eingeführt werden kann oder aus dieser herausgeführt werden kann.

Fig. 4 die Schlauchklemmvorrichtung 10 aus Fig. 3 im Querschnitt. Hierin werden weitere Unterschiede zu der bekannten Schlauchdurchführung 10 aus den Figuren 1 und 2 deutlich. In der erfindungsgemäßen Schlauchdurchführung 10 der Figuren 3 und 4 sind die Elektromagneten 26 ähnlich aufgebaut wie in den anderen Formen der Figuren 1 und 2, jedoch ist in diesem Fall der Anker 28 auf der Unterseite gegenüber dem Lager 35 mit einer Klemmfeder 34 federvorbelastet, die den Anker 28 im unbestromten Zustand der Magnetspule 30 aus der Magnetspule 30 heraus nach oben drückt. Im bestromten Zustand wird der Anker 28 in die Magnetspule 30 hineingezogen, bis sie den Anschlag, also die Oberseite des Lagers 35, kontaktiert.

In den Anker 28 greift ein Bolzen 36 ein, dessen unteres Ende mit einer Mutter und einer ringförmigen Endlagerdämpfung 38 versehen ist, und welche einen Anschlag für die Bewegung des Ankers 28 nach oben in Klemmrichtung der Schlauchklemmvorrichtung 10 bildet. Der Bolzen 36 ist, beispielsweise über einen Splint oder eine Schraube, fest mit dem Anker 28 verbunden. Wird das untere Ende des Bolzens 36 gedreht, dreht sich der Anker 28 mit. Der Adapter 24 ist über eine Schraubverbindung mit dem Anker 28 verbunden. Zum Einstellen des Klemmspalts kann der Adapter 24 mittels eines geeigneten Instruments festgehalten werden, das zu diesem Zweck durch eine Öffnung im Gehäuse 16 geführt wird, während gleichzeitig durch Drehen des Bolzens 36 der Abstand des Adapters 24 zum Anker 28 verändert wird.

Im oberen Teil innerhalb des Gehäuses 16 sind weitere Unterschiede zu erkennen. So ist der Druckknopf 40 zentral mit einem Bolzen 42 verbunden, der an seiner Unterseite in eine Rückstellfeder 44 eintaucht, die den Druckknopf 40 mit einer Federkraft vorbelastet, die den Druckknopf 40 in seine obere Position drückt, in welcher die Schlauchdurchführung 12 seitlich verschlossen ist. Die Rückstellfeder 44 drückt sich in einem Lager 46 ab, welches außerdem als Anschlag für die Unterseite des Bolzens 42 dient. Sobald die Unterseite des Bolzens 42 diesen Anschlag berührt, lässt sich der Druckknopf 40 nicht weiter nach unten drücken. Ein oberer Anschlag ist mittels eines Rands 41 an der Unterseite des Druckknopf 40 vorhanden, welcher in einer oberen Position des Druckknopfs 40 gegen eine umlaufende Kante im Gehäuse 16 anschlägt.

Der Bolzen 42 verfügt außerdem über einen quer sitzenden Mitnehmer 48, welcher sich mit dem Bolzen 42 bzw. den Druckknopf 40 bewegt. Auf diesem Mitnehmer 48 sitzt ein Ende eines Hebels 50 mit einer länglichen Nut 54 auf. Der Hebel 50 ist auf der gegenüberliegenden Seite an seinem anderen Ende auf einem Gelenk 52 schwenkbar befestigt. Wenn der Druckknopf 40 betätigt wird, nimmt der Mitnehmer 48 somit den Hebel 50 mit, der dabei um das Gelenk 52 verschwenkt wird.

Diese Verschwenkung des Hebels 50 hat zur Folge, dass der Hebel 50 ein erstes Verbindungsteil 23A der Verbindung zwischen dem Anker 28 und dem Druckstück 20 kontaktiert und mit einer auf den Druckknopf 40 ausgeübten Kraft beaufschlagt. Dies führt dazu, dass, sobald eine ausreichende Kraft ausgeübt wird, der Anker 28 und damit auch das Druckstück 20 nach unten gedrückt werden, entgegen der Federkraft der Klemmfeder 34. Auf diese Weise wird eine Notöffnungsfunktion realisiert. Das Erreichen der Notöffnungsfunktion ist auch deutlich fühlbar, weil das Herunterdrücken des Druckknopfs 40 zunächst nur gegen die Rückstellkraft der Rückstellfeder 44 erfolgt, ab der Kontaktierung der Oberseite des Verbindungsstücks 23 an zusätzlich auch noch gegen die von der Klemmfeder 34 bereitgestellte Kraft, welche wesentlich größer ist als die Rückstellkraft der Rückstellfeder 44.

Die Verbindung zwischen dem Anker 28 und dem Druckstück 20 ist, anders als in der in den Figuren 1 und 2 gezeigten Vorrichtung, nicht starr. Vielmehr sitzt auf dem Anker 28 ein Adapter 24 auf, welcher von außen auch im eingebauten Zustand der Schlauchklemmvorrichtung 10 zugänglich ist und mit dem der Klemmspalt, also die Größe der Schlauchdurchführung 12 in der Klemmposition eingestellt werden kann. Es kann sich bei dem Adapter 44 beispielsweise um eine Klemmvorrichtung auf einem Bolzen oder um eine Mutter mit Innengewinde auf einem Bolzen mit Außengewinde handeln, der mit dem Anker 28 verbunden ist.

Auf dem Adapter 24 sitzt im Ausführungsbeispiel ein Verbindungsstück 23 auf, das einstückig mit dem Druckstück 20 ausgebildet ist und die ersten und zweiten Verbindungsteile 23A, 23B umfasst. Das Verbindungsstück 23 weist eine Durchführung für den Hebel 50 auf und kann mit federnden Elementen gebildet werden und eine zusätzliche Flexibilität einführen. Es kann sich allerdings auch um separate Verbindungsteile 23A, 23B handeln, die aufeinander aufsitzen und auf diese Weise für Flexibilität sorgen. Auf die Oberseite des ersten Verbindungsteils 23A drückt der Hebel 50 bei Betätigung der Notöffnungsfunktion. Die Lücke zwischen dem ersten Verbindungsteil 23A und dem Druckstück 20 überbrückt ein zweites Verbindungsteil 23B, welches die Durchführung für den Hebel 50 bildet. Dieses kann mit federnden Elementen gebildet sein, welche einerseits eine Öffnung zur Durchführung des Hebels 50 offen lassen, und andererseits eine Flexibilität in die Verbindung zwischen dem Anker 28 und dem Druckstück 20 einführen So kann das zweite Verbindungstück 23B so ausgebildet sein, dass es sich bei Überschreiten eines oberen Druckgrenzwerts verbiegt und so die überschüssige Kraft aufnimmt, die zu einer Beschädigung eines Schlauchs führen könnte, was insbesondere der Fall sein kann, wenn die Einstellung des Adapters 24 in Bezug auf die Größe eines aktuell angelegten Schlauchs nicht korrekt ist und der Klemmspalt zu klein wird. Auf diese Weise kann der eingelegte Schlauch weiter geschützt werden.

Bei diesem Ausführungsbeispiel einer Schlauchklemmvorrichtung 10 werden die drei Betriebszustände Einlegen des Schlauches, Normalbetrieb und Notbetrieb so realisiert, dass zum Einlegen des Schlauches der Klemmspalt der Schlauchdurchführung 12 durch Bestromung der Magnetspule 30 des Elektromagneten 26 geöffnet wird, wodurch der Anker 26 einen Hub entgegen der Kraft der Klemmfeder 34 verrichtet. Durch die händische Betätigung des Druckknopfs 40, entgegen der Rückholfeder 44 des Druckknopfs 40, wird die Schlauchdurchführung 12 freigegeben und kann der Schlauch seitlich in die Schlauchdurchführung 12 eingelegt werden. Sobald der Druccknopf 40 nicht mehr gedrückt wird, verfährt er in seine obere Endposition und verhindert, dass der Schlauch ungewollt aus der Schlauchdurchführung 12 fallen kann.

Während des Normalbetriebs wird der Klemmspalt der Schlauchdurchführung 12 zwischen Druckstück 20 und Gegenlager 14 entsprechend dem gewünschten Anwendungsfall geöffnet und geschlossen, wodurch der eingelegte Schlauch geöffnet oder geklemmt wird. Das Öffnen erfolgt über das Bestromung der Magnetspule 30, wodurch eine Kraft auf den Anker 28 ausgeübt wird und dieser in Verbindung mit dem Druckstück 20 den Hub vollzieht. Beim Schlie-βen handelt es sich um eine sicherheitsrelevante Funktion. Das Schließen erfolgt im unbestromten Zustand mechanisch über die Klemmfeder 34 und ist somit unabhängig von der Energieversorgung. Über die Endlagendämpfung 38 wird der Geräuschpegel reduziert. Das Druckstück 20 ist über den Adapter 24 und die Verbindungsteile 23A, 23B mit dem Anker 28 mechanisch voneinander entkoppelt verbunden. Die spezielle mechanische Entkopplung kann Schiefstände des Druckstückes 20 ausgleichen, überbestimmte Lagerzustände verhindern, Reibung reduzieren und die Lebensdauer der Schlauchklemmvorrichtung 10 signifikant verlängern.

Im Falle eines Stromausfalls setzt der Notbetrieb ein. Der Anker 28 fällt ab und der Schlauch wird zwischen Gegenlager 14 und Druckstück 20 durch die Kleinfeder 34 sicher geklemmt. Soll der Schlauch in diesem Zustand entnommen werden, kann über eine erhöhte händische Kraft auf den Druckknopf 40 die resultierende Kraft über den Hebel 50 auf das Druckstück 20 übertragen werden. Die Schlauchklemmvorrichtung 10 öffnet sich. Weiterhin öffnet sich die seitliche Öffnung der Schlauchdurchführung 12 und der Schlauch kann entnommen werden.

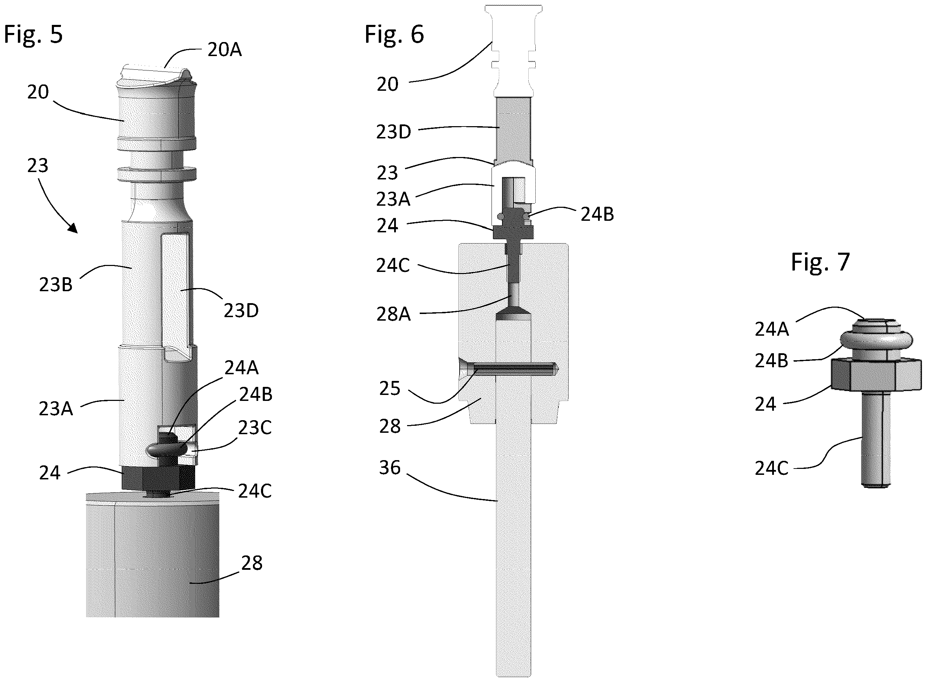

In Fig. 5 sind Einzelheiten der Verbindung zwischen dem Anker 28 und dem Druckstück 20 in einer perspektivischen Ansicht schematisch dargestellt. Auf dem Anker 28 befindet sich der Adapter 24, dessen Basis im vorliegenden Ausführungsbeispiel die Form eines Sechskants hat, im Wesentlichen wie eine Schraubenmutter. Nach unten hin weist der Adapter 24 ein längliches unteres Adapterteil 24C mit Außengewinde (nicht dargestellt) auf, welches in eine entsprechende Öffnung mit Innengewinde in der Oberseite des Ankers 28 eingeschraubt ist. Durch Drehen des Adapters 24 relativ zum Anker 28 kann der Abstand der Basis des Adapters 24 zum Anker 28 verändert und eingestellt werden.

Auf der Oberseite der Basis des Adapters 24 sitzt das Verbindungsstück 23 auf, welches in dem vorliegenden Ausführungsbeispiel einstückig mit dem Druckstück 20 ausgebildet ist, welches an seiner Oberseite einen Klemmkeil 20A aufweist, welches zum Klemmen eines Schlauchs dient. Das Verbindungsstück 23 ist längserstreckt und weist entlang seiner Längserstreckung mehrere Abschnitte auf. Oberhalb des Adapters 24 befindet sich ein erstes Verbindungsteil 23A, welches parallel zu seiner unteren Kante eine ringförmige Innennut 23C aufweist. Mit dem unteren Teil sitzt das Verbindungsstück 23 auf der Basis des Adapters 24 auf. Der Adapter 24 weist an seiner Oberseite ein kurzes oberes Adapterteil 24A auf, welches auf einer (nicht dargestellten) ringförmigen Nut einen O-Ring 24B aufweist, welcher an seiner Außenseite in die ringförmige Innennut 23C des ersten Verbindungsteils 23A eingreift. Über den O-Ring 24B wird die Zugkraft auf das Verbindungsstück 23 übertragen, wenn die Magnetspule 30 des Elektromagneten 26 bestromt wird und der Anker 24 zurückgeholt wird, um den Klemmspalt zu öffnen. Beim Schließen des Klemmspalts drückt die Basis des Adapters 24 direkt auf die Unterseite des Verbindungsstücks 23. In einem zweiten Verbindungsteil 23B zwischen dem ersten Verbindungsteil 23A und dem Druckstück 20 befindet sich eine Durchgangsöffnung 23D, durch welche der in Fig. 4 dargestellte Hebel 50 geführt wird.

In Fig. 6 sind einige der zentralen Komponenten der Schlauchklemmvorrichtung 10 im Querschnitt dargestellt. Hierin ist zu erkennen, dass der Bolzen 36 über einen Splint 25 mit dem Anker 28 des Elektromagneten verbunden ist. Der Bolzen 36 reicht bis ungefähr zur Hälfte des Ankers 28 in diesen hinein. Oberhalb des Bolzens verengt sich die Aufnahme, die im unteren Teil den Bolzen 36 aufnimmt, auf den Durchmesser des unteren Adapterteils 24C des Adapters 24 und weist ein Innengewinde auf, zu dem das Außengewinde auf dem unteren Adapterteil 24 C passt. So wird der Adapter 24 in die Aufnahme im Anker 28 eingeschraubt und kann zum Einstellen der Größe des Klemmspalts durch Herein- und Herausschrauben in seinem Abstand zum Anker 28 eingestellt werden. Dazu kann entweder der Adapter 24 selbst oder der Anker 28 um seine Achse gedreht werden.

Eine perspektivische Darstellung des Adapters 24 mit dem auf dem oberen Adapterteil 24A ausgestreckten O-Ring 24B ist schematisch in Fig. 7 gezeigt. Die sechskantige Form der Basis des Adapters 24 ist geeignet, beispielsweise von einem Schraubschlüssel erfasst und gehalten zu werden, wenn der Anker 28 gedreht wird, um den Abstand der Basis des Adapters 24 zum Anker 28 einzustellen.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 10: Schlauchklemmvorrichtung
- 12: Schlauchdurchführung
- 14: Gegenlager
- 15: Aussparung
- 16: Gehäuse
- 18: Notentriegelungshebel mit Exzenter
- 19: Scharnier
- 20: Druckstück
- 20A: Klemmkeil
- 21: Mitnehmerbolzen
- 22: Druckstange
- 23: Verbindungsstück
- 23A: erstes Verbindungsteil
- 23B: zweites Verbindungsteil
- 23C: ringförmige Innennut
- 23D: Durchgangsöffnung
- 24: Adapter
- 24A: oberes Adapterteil
- 24B: O-Ring
- 24C: unteres Adapterteil
- 25: Splint
- 26: Elektromagnet
- 28: Anker
- 28A: zentrale Bohrung
- 30: Magnetspule
- 32: Joch
- 34: Klemmfeder
- 35: Lager
- 36: Bolzen
- 38: Endlagendämpfung
- 40: Druckknopf
- 41: Rand
- 42: Bolzen
- 44: Rückstellfeder
- 46: Lager
- 48: Mitnehmer
- 50: Hebel
- 52: Gelenk
- 54: Nut

## Patentansprüche

1. Schlauchklemmvorrichtung (10) mit einem Gehäuse (16) und einer außerhalb des Gehäuses (16) angeordneten und in ihrem Querschnitt zwischen einer Klemmposition und einer geöffneten Position reversibel veränderbaren Schlauchdurchführung (12), die von einem fixen Gegenlager (14) und einem gegenüber dem fixen Gegenlager (14) verschiebbaren Druckstück (20) gebildet wird, welches mit einem in Richtung auf die Klemmposition federvorbelasteten Anker (28) eines Elektromagneten (26) verbunden ist, welcher im bestromten Zustand des Elektromagneten (26) das Druckstück (20) entgegen der Federvorbelastung vom Gegenlager (14) entfernt und dadurch die Schlauchdurchführung (12) aus der Klemmposition in die geöffnete Position bringt, **dadurch gekennzeichnet, dass** das Druckstück (20) mit dem Anker (28) über einen Adapter (24) verbunden ist, mittels dessen eine in der Klemmposition der Schlauchdurchführung (12) eingenommene Spaltbreite zwischen Druckstück (20) und dem fixen Gegenlager (14) einstellbar ist.

2. Schlauchklemmvorrichtung (10) nach Anspruch 1, wobei der Adapter (24) im montierten Zustand der Schlauchklemmvorrichtung (10) einstellbar und insbesondere durch eine Öffnung im Gehäuse (16) zugänglich ist.

3. Schlauchklemmvorrichtung (10) nach Anspruch 1 oder 2, wobei das Druckstück (20) mit dem Anker (28) flexibel verbunden ist und/oder vom Anker (28) entkoppelt ist.

4. Schlauchklemmvorrichtung (10) nach einem der Ansprüche 1 bis 3, wobei das fixe Gegenlager (14) auf einer Seite der Schlauchdurchführung (12) mit dem Gehäuse (16) verbunden ist und zur anderen Seite eine seitliche Öffnung zum Einfädeln oder zur Entnahme eines zu klemmenden Schlauchs aufweist, wobei an der geöffneten Seite der Schlauchdurchführung (12) ein Druccknopf (40) angeordnet ist, welcher in einem Ruhezustand die seitliche Öffnung der Schlauchdurchführung (12) verschließt und in einem um einen Hubweg gedrückten Zustand freigibt.

5. Schlauchklemmvorrichtung (10) nach Anspruch 4, wobei der Druckknopf (40) mit einem im Inneren des Gehäuses (16) angeordneten Hebel (50) an einem ersten Ende des Hebels (50) verbunden ist, welches sich mit dem Hub des Druckknopfs (40) mitbewegt, während ein zweites Ende des Hebels (50) mit einem Gelenk (52) im Gehäuse (15) verbunden ist, wobei der Hebel (50) bei Betätigung des Druckknopfes (40) im unbestromten Zustand des Elektromagneten (26) den Anker (28) unmittelbar oder mittelbar kontaktiert und entgegen der Federvorbelastung des Ankers (28) drückt, um die Schlauchdurchführung (12) in die geöffnete Position zu bewegen.

6. Schlauchklemmvorrichtung (10) nach Anspruch 4 oder 5, wobei der Druckknopf (40) in der Richtung der seitlichen Ausdehnung der Schlauchdurchführung (12) eine Breite aufweist, die größer ist als ein maximaler Abstand zwischen einer Klemmkontaktseite des Druckstücks (20) und einer der Klemmkontaktseite des Druckstücks (20) gegenüberliegenden Klemmkontaktseite des fixen Gegenlagers (14).

7. Schlauchklemmvorrichtung (10) nach einem der Ansprüche 4 bis 6, wobei der Druckknopf (40) durch eine Rückstellfeder (44) in eine die seitliche Öffnung der Schlauchdurchführung (12) verschließende Position federvorbelastet ist.

8. Schlauchklemmvorrichtung (10) nach einem der Ansprüche 1 bis 6, wobei die Federvorbelastung des Ankers (28) durch eine Klemmfeder (34) hergestellt ist, die auf der dem Druckstück (12) abgewandten Seite des Ankers (28) angeordnet ist.

9. Verfahren zum Bedienen einer Schlauchklemmvorrichtung (10) nach einem der Ansprüche 1 bis 8, **dadurch gekennzeichnet, dass** eine minimale Größe eines Spalts zwischen dem Druckstück (20) und dem fixen Gegenlager (14) der Schlauchklemmvorrichtung mittels des Adapters (24) eingestellt wird.

10. Verfahren nach Anspruch 9, wobei der Druckknopf (40) zum Freigeben der seitlichen Öffnung der Schlauchdurchführung (12) betätigt und zum Verschließen der seitlichen Öffnung losgelassen wird.

11. Verfahren nach Anspruch 10, wobei zum Notöffnen der Schlauchdurchführung (12) der Druckknopf (40) weiter betätigt wird, als es zum Freigeben der seitlichen Öffnung der Schlauchdurchführung (12) notwendig ist.
